# EUROPÄISCHE PATENTANMELDUNG

(11) **EP 1 702 918 A2**
(43) Veröffentlichungstag der Anmeldung: **20.09.2006**
(21) Anmeldenummer: 06100032.9
(22) Anmeldetag: 03.01.2006
(51) Int. Cl.: C07D 285/125

(54) **Verfahren zur Herstellung von Bis-DMTD (5,5-Dithio-bis-(1,3,4-thiadiazol-2-thiol))**

(30) Priorität: 31.01.2005 DE 102005004472
(71) Anmelder: RHEIN-CHEMIE RHEINAU GmbH, 68219 Mannheim (DE)
(72) Erfinder: Wühr, Michael, 69493, Hirschberg (DE); Laufer, Wilhelm, 68165, Mannheim (DE); Galda, Patrick, 76149, Karlsruhe (DE); Lemmin, Walter, 68623, Lampertheim (DE)
(74) Vertreter: Gille Hrabal Struck Neidlein Prop Roos

(57) **Zusammenfassung**

Die vorliegende Erfindung betrifft ein neues, verbessertes Verfahren zur Herstellung von Bis-DMTD (5,5'-Dithiobis-(1,3,4-thiadiazol-2-thiol)).

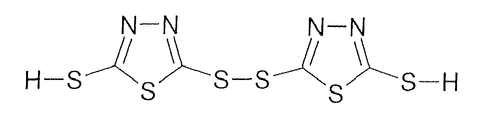

## Beschreibung

Die Erfindung betrifft ein neues Verfahren zur Herstellung von Bis-DMTD (5,5'-Dithiobis-(1,3,4-thiadiazol-2-thiol) der Formel (1) 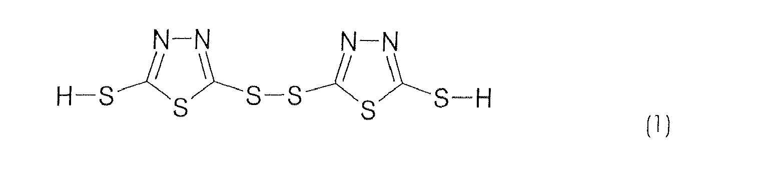 (im Folgenden meist nur Bis-DMTD genannt).

Bis-DMTD ist eine bekannte Verbindung, die insbesondere in Schmiermitteln verwendet wird, um den Schmiermitteln Extremdruckeigenschaften zu verleihen (vgl. insbesondere EP 0 122 317).

Als Verfahren zur Herstellung von Bis-DMTD erwähnt die EP 0 122 31 7 das Verfahren der Oxidation von 2,5-Dimercapto-1,3,4-thiadiazol (DMTD) mit Jod, wie es in der US 3 161 575 offenbart ist. Dieses Verfahren ist jedoch technisch äußerst nachteilig, da es den Einsatz des höchst korrosiven Jods erfordert, Außerdem wird bereits in besagter US 3 161 575 erwähnt, dass diese Herstellweise zur Bildung weiterer Produkte führt, bei denen es sich um polymere Oxidationsprodukte der Formel 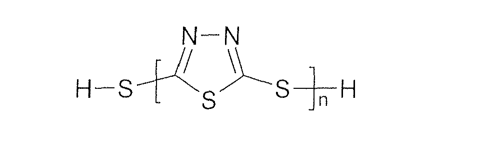 handelt, worin n > 1 ist, Diese polymeren Oxidationsprodukte müssen aufwändig abgetrennt werden, da ihre Anwesenheit die weiteren Anwendungen des gewünschten Dimers nachteilig beeinflusst.

Dieses bekannte Herstellverfahren geht zurück auf E. Ziegele, J. Prakt, Chem, 16, 40 (1899), der die Herstellung des Dimers durch Oxidation des Thiodiozol-2,5-dithiols in alkoholischen Lösungen mit Jod oder Eisen(III)chlorid beschreibt, Auch die Verwendung von Eisen(III)chlorid ist jedoch aufgrund der Anwesenheit von Halogeniden nachteilig, Weiterhin entsteht ein unlöslicher Rückstand, bei dem es sich wahrscheinlich ebenfalls um das oben gezeigte polymere Oxidationsprodukt handelt,

S, M, Losanitch in Soc. 121, 1542 (1921) führt die Oxidation in einer alkoholischen Lösung von DMTD mit einem Überschuss von Jod durch. Er erhält eine Verbindung, die nach seinen Angaben nicht identisch ist mit der von Ziegele gefundenen Polymerverbindung und die Zusammensetzung C₄H₄N₄S₇ hat.

Aus der EP 0 135 152 ist bekannt, dass die Umsetzung von 2,5-Di-mercapto-1 ,3,4-thiadiazol mit Wasserstoffperoxid zur Bildung eines dimeren Produktes der folgenden Formel führt (Bis-[2,5-dithio-1 ,3,4-thiodiozol]):

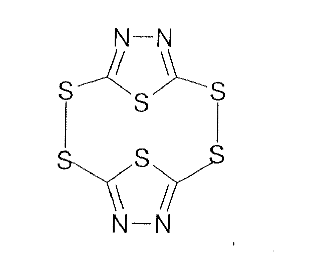

Die Oxidation von DMTD mit H₂O₂ ist bisher nur in Anwesenheit weiterer Reaktionspartner beschrieben worden. So werden in den US-Patenten 3 087 932 und 3 663 561 Verfahren beschrieben, bei denen durch Umsatz von DMTD, Wasserstoffperoxid und Mercaptanen öllösliche Derivate des DMTDs erhalten werden. Diese Produkte sind jedoch nicht einheitlich und bedürfen teilweise einer nachträglichen Aufarbeitung. Ein Verfahren zur Herstellung ähnlicher Verbindungen unter Verwendung von Chlor ist beispielsweise aus der US 3 821 236 bekannt.

Polymere des 1,2,4-Thiadiazols sind beispielsweise aus der US 4 107 059 bekannt,

Die Umsetzung von DMTD mit Wasserstoffperoxid führt gemäß US 4 246 126 zu einem nicht näher charakterisierten Material.

In der US 5 563 240 ist Umsetzung von DMTD mit Dichlorsulfanen in Gegenwart von Natronlauge beschrieben worden.

Ein für industrielle Maßstäbe zufriedenstellendes Verfahren zur Herstellung von Bis-DMTD steht daher bislang nicht zur Verfügung,

Die vorliegenden Erfinder stellten sich daher die Aufgabe, ein industriell zufriedenstellendes Verfahren zur Herstellung von Bis-DMTD ausgehend von DMTD zu finden, das in hohen Ausbeuten und hoher Selektivität zur Bildung von Bis-DMTD führt, Das Verfahren sollte insbesondere auch vorteilhaft auf den Einsatz hochkorrosiver hologenhaltiger Oxidationsmittel verzichten,

Diese Aufgabe kann überraschend gelöst werden durch Austausch der Schutzgruppe X in einem Bis-DMTD-Derivat der folgenden Formel (2) 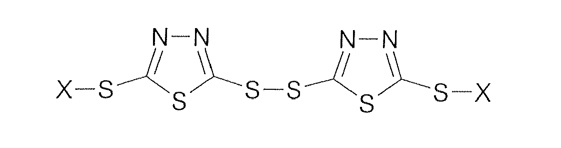 gegen Wasserstoff, vorzugsweise unter Verwendung einer Bronstedt-Säure. Bei der Gruppe X handelt es sich dabei im Prinzip um eine beliebige Schutzgruppe, die geeignet ist, die Mercaptogruppe im vor weiterer Oxidation mit einem nichthalogenhaltigen Oxidationsmittel, insbesondere durch Peroxide, unter Bildung einer Dithiogruppe (-S-S-) (mithin polymerer Nebenprodukte) zu schützen, und die beispielsweise durch saure Hydrolyse wieder in eine Mercaptogruppe überführt werden kann, Diese Voraussetzungen erfüllt überraschend insbesondere ein Alkalimetall, wie Natrium, Kalium, etc, Besonders bevorzugt handelt es sich bei der Gruppe X um Natrium. Es handelt sich dann im wesentlichen um eine salzartige Thio-Verbindung (-S M⁺, worin M ein Metall ist), Die Herstellung der Verbindung der Formel (2) gelingt insbesondere durch Umsetzung einer monomeren Verbindung der Formel (3) 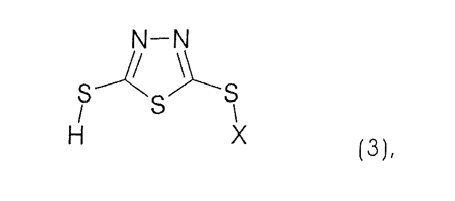 worin X wie oben definiert ist, besonders bevorzugt durch Umsetzung der Verbindung der Formel (3'): 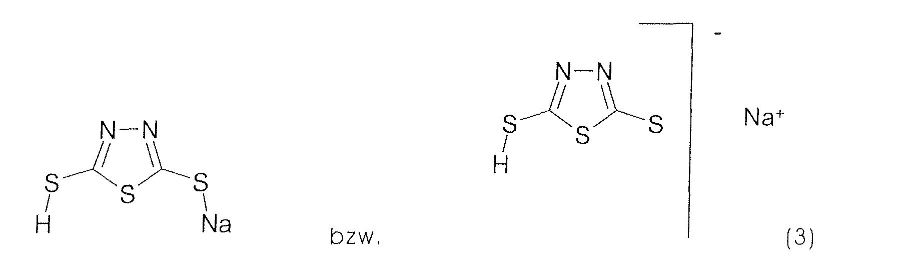 mit mindestens einem Peroxid, Überraschend unterliegt dabei die geschützte Mercaptogruppe -S-X, bzw, insbesondere -SNa, nicht der Oxidation, und es wird selektiv nur die -SH-Gruppe unter Bildung einer Dithiogruppe (-S-S-) oxidiert. Dadurch gelingt es, die Oxidation auf der Stufe der dimeren Verbindung der Formel (I) zu erhalten und die Verbindung der Formel (I) in hohen Ausbeuten und mit hoher Reinheit zu erhalten, Die vorliegende Erfindung betrifft daher insbesondere ein Verfahren zur Herstellung der Verbindung der Formel (I), das die folgenden Schritte umfasst:
(a) Umsetzen einer -SH-Gruppe in DMTD der Formel (4): 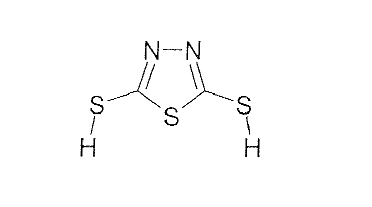 unter Bildung einer Verbindung der Formel (3) 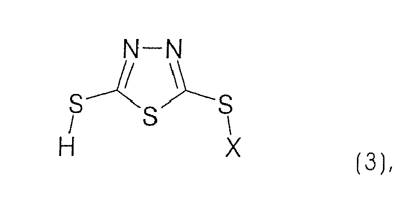 worin X eine Gruppe ist, die eine Oxidation des Schwefelatoms, an dem sie sich befindet, insbesondere durch weitere Umsetzung mit Peroxiden verhindert,
(b) Unterwerfen der Verbindung der Formel (3) mit mindestens einem nichthalogenhaltigen Oxidationsmittel, insbesondere einem Peroxid, unter Bildung einer Verbindung der Formel (2): 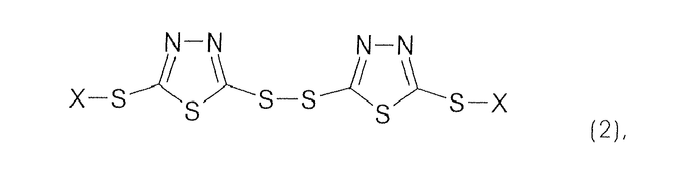 worin X wie oben definiert ist, und
(c) Überführen der Verbindung der Formel (2) in eine Verbindung der Formel (1): 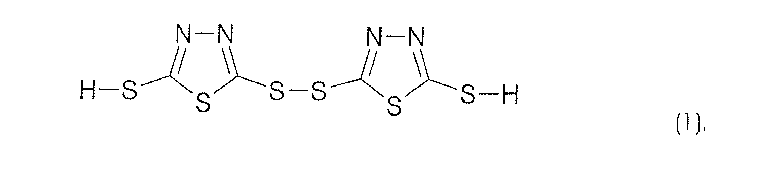 Bei dem Peroxid kann es sich um beliebige Peroxidverbindungen handeln, wie organische Peroxide oder anorganische Peroxide. Besonders bevorzugt handelt es sich jedoch um Wasserstoffperoxid. Wasserstoffperoxid wird bevorzugt in Form einer etwa 30 bis 40 %igen, insbesondere 35-%igen H₂O₂-Lösung verwendet, Die Umsetzung der Verbindung der Formel (3) mit H₂O₂ erfolgt bevorzugt bei leicht erhöhten Temperaturen (30 bis 80, bevorzugt 50 bis 60 °C) in wässriger Lösung,

Die Herstellung der Verbindung der Formel (3) gelingt in vorteilhafter Weise durch Umsetzung von DMTD mit äquimolaren Mengen einer die Schutzgruppe einführenden Verbindung, Bei der die Schutzgruppe einführenden Verbindung handelt es sich besonders bevorzugt um eine anorganische Lauge, wie insbesondere Natronlauge, Indem das molare Verhältnis von etwa 1 : 1 eingehalten wird, kann selektiv die mono-geschützte Verbindung der Formel (3) erhalten werden, Die Einhaltung dieses stöchiometrischen Verhältnisses kann in industriellem Maßstab durch einfache potentiometrische Messungen überwacht werden.

Gegenstand der Erfindung sind daher auch die Verbindungen der Formel (2) 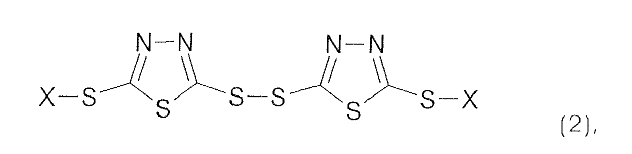 und der Formel (3) 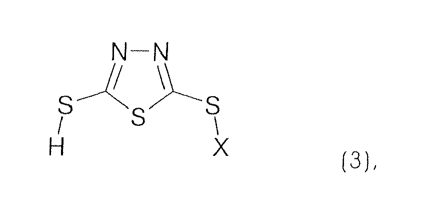 worin X jeweils wie oben definiert ist. Bevorzugt ist X ein Alkalimetall, besonders bevorzugt Natrium.

Die Herstellung der Verbindung der Formel (3) erfolgt besonders bevorzugt in Wasser oder einer Mischung von Wasser mit Alkoholen, wie z, B. Methanol oder Ethanol.

Da es sich bei der geschützten Verbindung der Formel (3) bevorzugt um ein Alkalimetallsalz handelt, ist diese Verbindung in Wasser bzw. wässrigen Lösungen löslich und die resultierende klare Lösung kann anschließend der Oxidation bevorzugt mit einem Peroxid unterworfen werden,

Die vorliegende Erfindung wird durch nachfolgendes Beispiel weiter erläutert:

### Beispiel:

In 400 ml Wasser werden 65,1 g (0,5 Mol) DMTD über 30 Minuten eingerührt, Nachfolgend werden 80,0 g einer 25 gew.-%igen Natronlauge (entsprechend 0,5 Mol) unter Wasserkühlung zudosiert. Nach der Zudosierung wird noch etwa eine Stunde bei Raumtemperatur nachgerührt, Die Bildung des Mono-Salzes wird durch Bestimmung der Säurezahl kontrolliert, Gegebenenfalls kann noch DMTD nachdosiert werden, Zu der erhaltenen Lösung wird eine 35 gew.-%ige Wasserstoffperoxidlösung (12,1 g) während etwa 30 Minuten gegeben. Es wird dann noch halbe Stunde nachgerührt. Nach Zugabe des Wasserstoffperoxids kann unmittelbar mit der Zugabe von Salzsäure (0,32 Gew.-%) Lösung, 28,5 g) begonnen werden, Das gewünschte Produkt Bis-DMTD fällt dabei als gelbes Pulver praktisch rein aus, Es wird abfiltriert und gewaschen, bis das Filtrat neutral reagiert und anschließend im Rotationsverdampfer getrocknet.

Die Ausbeute betrug > 95 % d. Theorie, und die spektroskopischen Eigenschaften entsprechen denen des bekannten Produktes,

## Patentansprüche

1. Verfahren zur Herstellung von Bis-DMTD (5,5-Dithio-bis-(1,3,4-thiadiazol-2-thiol)) der Formel (1) 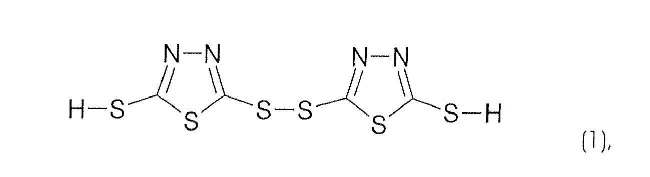 welches den Schritt (c) umfasst:
Überführen der Gruppe X, in einer Verbindung der Formel (2) 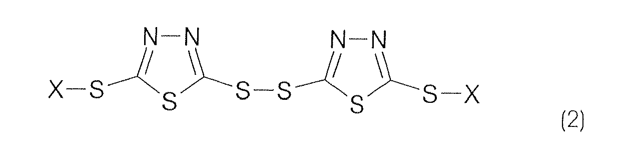 worin X eine Schutzgruppe darstellt, in ein Wasserstoffatom unter Bildung von Bis-DMTD der Formel (1).

2. Verfahren nach Anspruch 1, das die Schritte umfasst:
(b) Unterwerfen einer Verbindung der Formel (3) 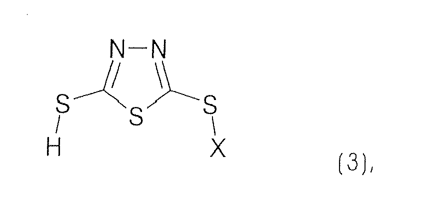 worin X eine Schutzgruppe darstellt, der Oxidation mit mindestens einem Oxidationsmittel unter Bildung der Verbindung der Formel (2): 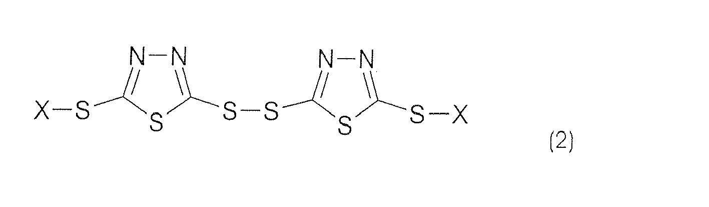 worin X wie oben definiert ist, und
(c) Überführen der Gruppen X in der Verbindung der Formel (2) 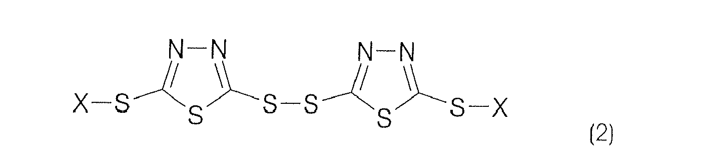 worin X wie oben definiert ist, in Wasserstoffatome unter Bildung von Bis-DMTD der Formel (1).

3. Verfahren nach Anspruch 2 worin das Oxidationsmittel kein Halogen enthalt.

4. Verfahren nach Anspruch 2 oder 3 worin das Oxidationsmittel aus Peroxiden ausgewählt wird,

5. Verfahren nach Anspruch 4, worin das Peroxid aus anorganischen oder organischen Peroxiden ausgewählt wird.

6. Verfahren nach einem der Ansprüche 2 bis 5, worin das Oxidationsmittel Wasserstoffperoxid bzw, eine wässrige Lösung davon ist.

7. Verfahren nach einem der Ansprüche 1 bis 6, das die Schritte umfasst;
(a) Einführung einer Schutzgruppe X in eine Verbindung der Formel (4): 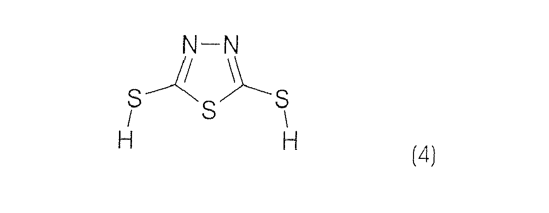 unter Bildung einer Verbindung der Formel (3) 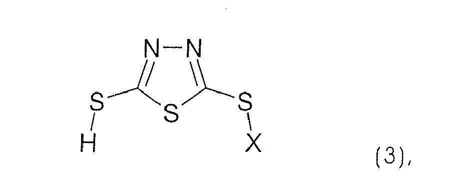 worin X eine Schutzgruppe darstellt,
(b) Unterwerfen einer Verbindung der Formel (3) 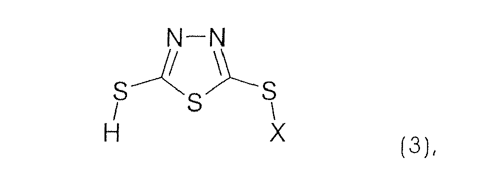 worin X wie oben definiert ist, der Oxidation mit mindestens einem Oxidationsmittel unter Bildung der Verbindung der Formel (2): 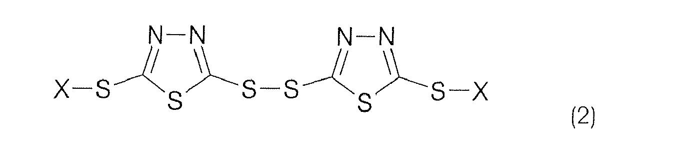 und
(c) Überführen der Gruppen X in der Verbindung der Formel (2) 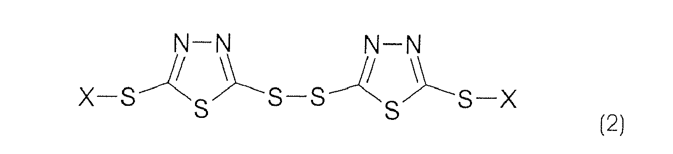 worin X wie oben definiert ist, in Wasserstoffatome unter Bildung von Bis-DMTD der Formel (1).

8. Verfahren nach einem der Ansprüche 1 bis 7, worin X eine Schutzgruppe ist, die die Gruppe -S-X vor der Oxidation mit einem Oxidationsmittel, wie insbesondere einem Peroxid schützt,

9. Verfahren nach einem der Ansprüche 1 bis 8, worin X ein Metall ist.

10. Verfahren nach einem der Ansprüche 1 bis 9, worin X ein Alkalimetall ist.

11. Verfahren nach einem der Ansprüche 1 bis 1 0, worin X Natrium ist,

12. Verfahren nach einem der Ansprüche 1 bis 1 1 , worin im Schritt (c) das Überführen der Schutzgruppe X in das Wasserstoffatom durch saure Hydrolyse erfolgt.

13. Verfahren nach einem der Ansprüche 1 bis 12, worin im Schritt (c) das Überführen der Schutzgruppe X in das Wasserstoffatom durch Behandlung mit einer Mineralsäure erfolgt,

14. Verbindung der Formel (2) 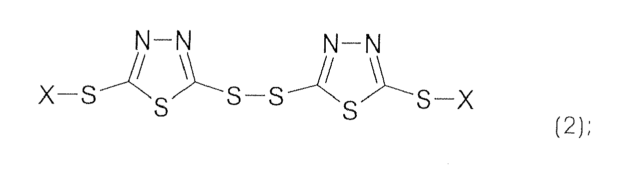 worin X eine Schutzgruppe ist, die geeignet ist, das Schwefelatom, mit dem sie verbunden ist, vor der Oxidation mit einem Oxidationsmittel, wie insbesondere einem Peroxid zu schützen.

15. Verbindung der Formel (3) 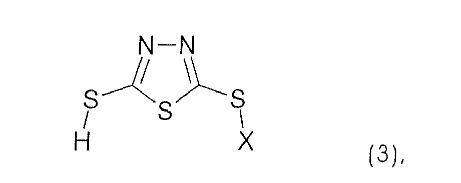 worin X eine Schutzgruppe ist, die geeignet ist, das Schwefelatom, mit dem sie verbunden ist, vor der Oxidation mit einem Oxidationsmittel, wie insbesondere einem Peroxid zu schützen.

16. Verbindungen nach Anspruch 14 oder 15, worin X ein Metall ist.

17. Verbindungen nach einem der Ansprüche 14 bis 1 6, worin X ein Alkalimetall ist.

18. Verbindungen nach einem der Ansprüche 14 bis 17, worin X Natrium ist.

19. Verwendung der Verbindungen der Formel (2) oder (3) für die Herstellung von Bis-DMTD (5,5'-Dithiobis-(1,3,4-thiadiazol-2-thiol) der Formel (1)
